# EUROPÄISCHE PATENTSCHRIFT

(11) **EP 0 740 547 B1**
(45) Veröffentlichungstag und Bekanntmachung des Hinweises auf die Patenterteilung: **24.02.1999**
(21) Anmeldenummer: 95906969.1
(22) Anmeldetag: 18.01.1995
(51) Int. Cl.: A61K 9/127, A61K 38/00, A61K 38/04, A61K 38/21, A61K 38/43

(54) **LIPOSOMEN ENTHALTEND DARIN VERKAPSELTE PROTEINE, VERFAHREN ZU IHRER HERSTELLUNG SOWIE DIESE LIPOSOMEN ENTHALTENDE PHARMAZEUTISCHE UND KOSMETISCHE ZUBEREITUNGEN**
LIPOSOMES CONTAINING ENCAPSULATED PROTEINS, METHOD OF PREPARING THEM AND PHARMACEUTICAL AND COSMETIC PREPARATIONS CONTAINING SUCH LIPOSOMES
LIPOSOMES CONTENANT DES PROTEINES ENCAPSULEES, LEUR PROCEDE DE FABRICATION ET PREPARATIONS PHARMACEUTIQUES ET COSMETIQUES CONTENANT CES LIPOSOMES

(30) Priorität: 31.01.1994 DE 4402867
(43) Veröffentlichungstag der Anmeldung: 06.11.1996
(73) Patentinhaber: Dr. Rentschler Arzneimittel GmbH & Co., 88471 Laupheim (DE)
(72) Erfinder: SCHMIDT, Peter, Christian, D-72074 Tübingen (DE); KARAU, Christine, D-72070 Tübingen-Unterjesingen (DE); PETSZULAT, Monika, D-88471 Laupheim (DE); WALCH, Hatto, D-88471 Laupheim-Baustetten (DE)
(74) Vertreter: Schwabe - Sandmair - Marx
(86) Internationale Anmeldenummer: EP9500175
(87) Internationale Veröffentlichungsnummer: WO9520379

(56) Entgegenhaltungen:
- EP-A- 0 357 005
- EP-A- 0 432 693
- WO-A-86/06959
- WO-A-87/04592
- WO-A-88/01864
- WO-A-91/16882
- US-A- 4 241 046
- US-A- 4 752 425
- US-A- 4 933 121
- US-A- 5 049 392
- THE JOURNAL OF INFECTIOUS DISEASES, Band 159, Nr. 4, ver!ffentlicht April 1989, D.A. EPPSTEIN et al. "Liposomal Interferon- : Sustained Release Treatment of Simian Varicella Virus Infection in Monkeys", Seiten 616-620,

## Beschreibung

Die Erfindung betrifft Liposomen, die Proteine, vorzugsweise Interferone, darin verkapselt als Wirkkomponenten enthalten, wobei diese Proteine mit einer hohen Einschlußrate verkapselt werden, Verfahren zu deren Herstellung sowie diese Liposomen enthaltende pharmazeutische und kosmetische Zubereitungen.

Verschiedene Peptidhormone oder Proteine sind in letzter Zeit als therapeutisch wirksame Mittel bei der Bekämpfung von Krankheiten wie Diabetes, Hormon- oder Wachstumsstörungen, Schilddrüsenerkrankungen aber auch viralen und/oder bakteriellen Infekten und Krebs eingesetzt worden. Solche Peptide sind beispielsweise Oxytocin, Vasopressin, FSH, TSH, LH, Tumor Nekrosis Faktoren, Growth Faktoren, Plättchen aktivierende Faktoren, Insulin, Calcitonin, Cyclosporin A, Interleukine und Interferone. In den meisten Fällen werden diese Proteine zur Therapie in Form von Lösungen injiziert. Ein weitreichendes Problem stellt dabei die Instabilität der Proteine sowohl in dieser Lösung als auch nach der Injektion in der Blutbahn dar.

In der Blutbahn werden die genannten Proteine schnell von endogenen Proteasen abgebaut und verlieren so ihre spezifische Wirkung. Andererseits stellen sie aufgrund ihrer Proteinnatur Antigene dar, die eine Immunantwort mit den entsprechenden Folgen bzw. Nebenwirkungen hervorrufen können. Trotz dieser Probleme und Nachteile ist man wegen der sehr spezifischen Wirkung der Peptidhormone und Proteine auf einzelne Zellgruppen oder Zellen auf ihren Einsatz bei der Therapie bei den genannten Krankheiten angewiesen. Im folgenden soll dies anhand des Beispiels der Interferone näher erläutert werden.

Interferone sind Proteine oder Glykoproteine, die auf äußere Reize hin von bestimmten Zellen (Leucozyten oder Fibroblasten) gebildet werden. Sie haben ein Molekulargewicht von ungefähr 20 kD, entsprechend 140 bis 160 Aminosäuren. Interferone zeichnen sich erstens durch eine antivirale Aktivität aus. Eine solche echte Bekämpfung von Viren ist aber mit herkömmlichen Antibiotika nicht möglich.

Interferone induzieren, vermittelt über Zelloberflächenrezeptoren, die Synthese von sogenannten "antiviralen Proteinen". Diese Proteine wiederum verhindern über verschiedene Mechanismen, z.B. Transkriptionshemmung oder Oligo A-Synthese und die dadurch bewirkte Stimulation der zellulären RNAse-Aktivität, die Reproduktion des Virus. Diese Wirkung ist artspezifisch. Darüber hinaus sind diese Interferone durch ihre wachstumshemmende Wirkung als Cytostatika in der Krebstherapie, z.B. der Haarzell-Leukämie, von Bedeutung. Durch Aktivierung von Makrophagen, T-Zellen und Killerzellen wird die Immunantwort stimuliert. Außerdem wird die Ausschüttung des Tumor-Nekrosis-Factors-α stimuliert, wodurch sich weitere pharmazeutisch interessante Anwendungsmöglichkeiten abzeichnen. Interferone steigern daneben die Antigenpräsentation der T-Zellen und aktivieren Makrophagen (antibakterielle Wirkung).

Bekannte Indikationen für die Gabe von Interferonen sind beispielsweise akute Viruserkrankungen wie Herpes zoster oder die Virusenzephalitis, chronische Viruserkraukungen wie die chronisch-aktive Hepatitis B, Kondylome, Warzen oder Zervixneoplasien, sowie Hämoblastosen wie die Haar-Zell-Leukämie, essentielle Thrombozythämie, chronisch lymphatische oder myeloische Leukämie, kutane T-Zell-Lymphome oder Plasmozytome. Darüber hinaus können Interferone bei der Therapie von anderen Karzinomen wie Karzinoid, Nasopharynxkarzinom, malignem Melanom oder Nierenzellkarzinom, verschiedensten endzündlichen Erkrankungen wie rheumatische Erkrankungen (chronische Polyarthritis), Verbrennungen, Erfrierungen, die atopische Dermatitis, Psoriasis, Sklerodermie aber auch zur Therapie der Multiplen Sklerose eingesetzt (vgl. Med.Mo.Pharm. 6, 1991, S.164-73).

Wie oben allgemein beschrieben, wurden die Proteine, d.h. auch die Interferone, als solche in pharmazeutischen Zubereitungen eingesetzt. Dabei wurden spezielle Anstrengungen unternommen, um diese zunächst in der zu injizierenden Lösung zu stabilisieren. Beispielsweise sind Interferon-stabilisierende Zubereitungen aus der DE-A-36 42 223 bekannt.

Zusätzlich wurde versucht, die Proteine nach der Injektion im Körper vor einem vorzeitigen Abbau durch körpereigene Proteasen zu schützen. Dazu werden die Proteine zum Beispiel in Liposomen verkapselt und so durch die zwischen dem Protein und der Protease liegende Lipidmembran(en) vor dem Abbau geschützt. Zu diesem Zweck wurden sowohl monolamellare als auch multilamellare Vesikel (Liposomen) eingesetzt. Bestimmende Faktoren der Geschwindigkeit, mit der das verkapselte Protein freigesetzt wird, sind, ohne sie vollständig anzugeben, die Größe des Vesikels, die Anzahl der Lamellen oder die Art der Komponenten, aus denen der Vesikel besteht, so US-A-5 0 23 087.

Liposomen, in denen ein pharmazeutischer Wirkstoff verkapselt ist, können aus einer oder mehreren Lipidkomponenten bestehen. So beschreibt US-A-5 023 087 den Einschluß von Proteinen in Liposomen aus Phosphatidylcholin (PC) und/oder Phosphatidylglycerol (PG)/Tocopherol, während die Verkapselung von Interferon in Liposomen aus Phosphatidylcholin und Phosphatidylserin (PS), meist in einem Mengenverhältnis von 7:3, in J.Interferon Res. 10(2), S.153-160, 1990 (β-IFN); J.Nat.Cancer Inst. 81(18), S.1387-92, 1989 (γ-IFN) oder J.Biol.Response Modif. 9(4), S.995-60, 1990 (α-IFN) sowie in EP-A 89 810 133 beschrieben ist.

Andere Lipidzusammensetzungen wie Phosphatidylglycerol/Cholesterol (Ch) (2:1) zum Einschluß von γ-IFN (Infect.Immun. 57(1), S.132-37, 1989 oder J.Infect.Dis. 159(4), S.616-20, 1989, Molverhältnis 9:1) und PC/Ch/Sulfatide (Molverhältnis 5:4:1) zum Einschluß von Interferonen (JP 6 228 393 4) sowie PC/PG/Ch und wenig Tocopherol (Molverhältnis ca. 11:8:1 in WO-A-91 16 882 oder ca. 10:8:1 in WO-A-87 04592) zur Verkapselung von nicht-Protein Pharmaka bzw. Proteinen werden ebenso verwendet.

Ein kritischer Faktor bei der Verwendung und Synthese von Liposomen ist die Einschlußrate der betreffenden Substanz in die Liposomen. Generell gilt, daß das Protein, um tatsächlich geschützt zu werden, komplett verkapselt sein muß und nicht lediglich mit der Vesikelmembran assoziiert oder in diese insertiert sein darf. Da viele Proteine neben hydrophilen auch hydrophobe Bereiche besitzen, ist ein solcher vollständiger Einschluß nicht selbstverständlich. Liegt die Einschlußrate sehr niedrig, so ist die Konzentration des eingeschlossenen Wirkstoffes sehr gering. WO-A-90 11780 beschreibt beispielsweise bei einem Verhältnis von PC/PG/Ch von 7:3:6 eine Einschlußrate von 6,9 %. Maximale Einschlußraten für Proteine in multilamellare Vesikel werden in WO-A-87 04592 mit 10 bis 20% beschrieben. Für nicht-Protein Pharmaka (Albuterolsulfat) werden dagegen mit Liposomen aus PC und PG im Verhältnis von 11:1 Einschlußraten von über 50 % angegeben. Solche hohen Einschlußraten sind für Proteine bislang nicht erreicht worden. Dementsprechend müssen große Mengen an Protein zur Synthese der Vesikel eingesetzt werden. Darüber hinaus müssen daher große Mengen an Substanz bei der Verabreichung von pharmazeutischen Zubereitungen, welche die Proteine verkapselt enthalten, appliziert werden. Dieses ist jedoch bei teuren oder schwer erhältlichen Wirkstoffen wie Peptidhormonen nicht erwünscht.

Es ist daher Aufgabe der vorliegenden Erfindung Liposomen, die Proteine mit einer hohen Einschlußrate verkapselt enthalten, ein Verfahren zu ihrer Herstellung sowie diese Liposomen enthaltende pharmazeutische und kosmetische Zubereitungen zur Verfügung zu stellen. Trotz der verbesserten Einschlußraten sollen die bekannten Vorteile der Verkapselung wie definierte Freisetzungsraten etc. nicht vermindert werden.

Überraschender Weise wurde gefunden, daß Einschlußraten von Proteinen in Liposomen durch den erfindungsgemäßen Einsatz von Phosphatidylglycerol in definiertem Mengenverhältnis mit Phosphatidylcholin und Cholesterol stark erhöht werden. Durch das erfindungsgemäße Verfahren zur Herstellung von Liposomen können Einschlußraten in die Liposomen von über 35 % gegenüber den bisher erzielbaren Raten, siehe oben, von unter 20 % erreicht werden.

Diese Steigerung der Einschlußraten ermöglicht es nun geringere Mengen an Protein zur Synthese einzusetzen und somit kostengünstiger zu produzieren. Darüber hinaus können, da mehr Wirkeinheiten pro Vesikel möglich sind, höhere Dosen des Therapeutikums appliziert oder das applizierte Volumen verringert werden.

Mit Hilfe des erfindungsgemäßen Verfahrens werden Liposomen und daraus pharmazeutische und Kosmetische Zubereitungen erhalten, die alle bekannten Vorteile von in Liposomen verkapselten Proteinen aufweisen. So wird das betreffende Protein definiert aus den Vesikeln freigesetzt und ist aufgrund der Verkapselung gegenüber proteolynschem Abbau geschützt. Da die Einschlußrate jedoch höher ist, ist dieser Schutz ausgeprägter als bei nach dem Stand der Technik erhaltenen Liposomen. Die diese Liposomen enthaltenden Zubereitungen sind dementsprechend stabiler. Außerdem verringern die erfindungsgemäßen Zubereitungen die Gefahr von immunologischen Nebenreaktionen, welche auf der antigenen Wirkung der Proteine beruhen, denn diese sind eben aufgrund der Verkapselung für die Antikörper in hohem Maße unzugänglich.

Die Erfindung betrifft demzufolge mit hoher Einschlußrate in Liposomen verkapselte Proteine sowie ein Verfahren zu deren Herstellung und pharmazeutische und kosmetische Zubereitungen, die solche Liposomen enthalten.

Die proteinhaltigen Liposomen werden aus Lipid, Cholesterol, einem Phosphatidylglycerol (PG) oder dessen Derivaten und gegebenenfalls Tocopherolen oder vergleichbaren Stabilisatoren wie Butylhydroxyanisol (BHA) oder Butylhydroxytoluol (BHT) in definiertem Mengenverhältnis über das unten beschriebene Verfahren gebildet.

In den erfindungsgemäßen Liposomen beträgt das molare Verhältnis von Lipid zu Cholesterol zu PG zu Tocopherol 6 : 4 : 1 : 0,01. Das Gewichtsverhältnis beträgt dementsprechend 6 : 2 : 1 : 0,01.

Grundsätzlich können alle Proteine für verschiedene Zwecke in die Liposomen eingeschlossen werden. Vorzugsweile handelt es sich dabei aber um pharmazeutisch wirksame Substanzen wie zum Beispiel Oxytocin, Vasopressin, FSH, TSH, LH, Tumor Nekrosis Faktoren, Growth Faktoren, Plättchen aktivierende Faktoren, Streptokinase, Urokinase, Insulin, Calcitonin, Cyclosporin A, Interteukine und Interferone. Bevorzugt werden Interferone, am meisten bevorzugt wird α-Interferon. Geeignete α-Interferone sind alle bekannten Subtypen des α-Interferons und deren Gemische sowie dessen Derivate, einschließlich aller Subtypen.

Geeignete Derivate des Phosphatidylglycerol, die in der Herstellung der erfindungsgemäßen Liposomen verwendet werden, sind dessen Mono- und Diester mit gesättigten und ungesättigten C₁₂₋₂₄-Fettsäuren. Vorzugsweise werden Palmitin-, Stearin-, Olein- und Myristinsäurediester eingesetzt.

Unter "Lipid" sind in diesem Zusammenhang Phosphatidylcholine, d.h. PC und dessen Fettsäureester mit den für das PG genannten Fettsäuren, zu verstehen, beispielsweise das kommerziell erhätliche Lipoid E 100 (Warenzeichen) und die Epicurone wie Epicuron 200 (Warenzeichen).

Außerdem wird ein Verfahren zur Herstellung von Liposomen mit einer hohen Einschlußrate für Proteine sowie zur Herstellung von pharmazeutischen und kosmetischen Zubereitungen, die diese Liposomen enthalten, zur Verfügung gestellt, welches die folgenden Schritte umfaßt:
a) Lösen der Liposomenanteile in einem geeigneten Lösungsmittel, Abziehen des Lösungsmittels in einem Rotationsverdampfer und Trocknen des entstandenen Lipidfilms,
b) Zugabe des einzuschließenden Proteins in einer pharmazeutisch geeigneten Lösung, Ablösen der Liposomen z.B. mittels Glaskugeln und anschließender Membranextrusion und
c) Überführen der in Schritt (b) erhaltenen Liposomen in eine pharmazeutisch oder kosmetisch geeignete Form, beispielsweise ein Gel, eine Creme, eine Lotion, eine Lösung oder ein Spray mittels bekannter Verfahren und unter Verwendung geeigneter Träger- und Hilfsstoffe.

Nach Verfahrensschritt (b) werden oligolamellare Liposomen mit einem Durchmesser von etwa 200 nm erhalten. Eine solche Liposomengröße ermöglicht eine definierte und kontrollierte Freisetzung der verkapselten Proteine, die nach dem erfindungsgemäßen Verfahren mit einer Einschlußrate von über 35 % verkapselt werden. Die nach dem Verfahren hergestellten Zubereitungen können somit höhere Dosen an Wirkstoff pro Vesikel enthalten und erlauben daher eine höhere Dosierung in der Anwendung oder die Applikation geringerer Mengen.

Darüber hinaus stabilisieren die erfindungsgemäßen Liposomen die darin verkapselten Proteine. Ihre biologische Aktivität bleibt im Vergleich mit in Liposomen des Standes der Technik eingeschlossenen Proteinen über einen längeren Zeitraum nahezu unverändert Daher weisen die pharmazeutischen und kosmetischen Zubereitungen, die die erfindungsgemäßen Liposomen enthalten, eine deutlich verbesserte lagerfähigkeit auf.

Geeignete Lösungsmittel zur Anwendung in dem erfindungsgemäßen Verfahren zum Lösen der Lipidkomponenten sind alle inerten organischen Lösungsmittel mit hohem Dampfdruck bei Raumtemperatur wie Chloroform, Methylenchlorid, Aceton, Methylethylketon, Hexan, Cyclohexan und dergleichen. Die genannte, pharmazeutisch geeignete Lösung ist eine gepufferte, isotonische Lösung. Bevorzugt ist eine Natriumphosphat-gepufferte, mit NaCl isotonisierte, Serumalbumin enthaltende Lösung.

In jeder genannten Zubereitung können die Liposomen neben dem verkapselten Protein weitere pharmazeutisch oder kosmetisch wirksame Substanzen eingeschlossen enthalten.

Zum Erhalt eines Gels werden bekannte Gelbildner eingesetzt, die in der Lage sind, ohne die Einwirkung hoher Scherkräfte, die zu einem weitgehenden Verlust der Proteinaktivität führen würden, Gele mit den Liposomen zu bilden. Solche Gelbildner stammen aus der Reihe der halbsynthetischen Cellulosederivate sowie der Alginate. Bevorzugt sind Natriumcarboxymethylcellulose, Hydroxyethylcellulose, Hydroxypropylcellulose, Hydroxypropylmethylcellulose sowie Natriumalginat. Cremes und Lotionen können entsprechend über bekannte Verfahren formuliert werden. Diese pharmazeutisehen und kosmetischen Zubereitungen können darüber hinaus alle bekannten Stabilisatoren, Antioxidantien, Pigmente, Geruchsstoffe und andere Hilfsstoffe enthalten, unter der Maßgabe, daß sie die Stabilität der Liposomen und der sie bildenden Komponenten nicht beeinträchtigen.

Erfindungsgemäße Zubereitungen in Form von Lösungen, die die oben erhaltenen Liposomen enthalten, werden gemäß dem beschriebenen Verfahren hergestellt, indem die Liposomen mit pharmazeutisch geeigneten, isotonisierenden Lösungen auf die gewünschte Konzentration gebracht werden. Pharmazeutisch und kosmetisch geeignete Lösungen sind mit einem verträglichen Puffer, wie Natriumphosphat oder Citrat, der auch die Stabilität des Proteins nicht beeinträchtigt, eingestellt und können mit Hilfe von NaCl, Polyolen, Zuckern oder Zuckeralkoholen isotonisiert sein. Diesen Lösungen können alle bekannten und üblichen Stabilisatoren, Antioxidantien, Pigmente, Additive, Proteine wie Serumalbumin, Zucker, Polyole und Zuckeralkohole zugesetzt werden unter der Voraussetzung, daß diese Zusätze die Stabilität der Liposomenkomponenten nicht beeinträchtigen.

Die erfindungsgemäß erhaltenen Zubereitungen können auf verschiedene Arten appliziert werden. In Form von Lösungen können sie auf verschiedenste Weise (i.v., i.p., i.c., s.c.) injiziert werden, so können sie z.B. bei der intratumoralen Injektion direkt in den Tumor eingespritzt werden. Dafür wird eine sterile Zubereitung benötigt, die durch aseptische Herstellung der Liposomen mit einer Sterilfiltration als Endstufe nach der Herstellung der Lösung erfolgen kann. Da die oligolamellaren Liposomen durch Extrusion durch Membranen vom Durchmesser 200 nm hergestellt werden können, ist die Sterilfiltration in der Endstufe dann ein zusätzlicher Schritt zur Qualitätsabsicherung.

Eine weitere mögliche Applikationsform für Lösungen sind Sprays, zur Verabreichung der in den Liposomen enthaltenen Wirkkomponente über die Haut oder Schleimhäute.

Außerdem können die pharmazeutischen und kosmetischen Zubereitungen epicutan appliziert werden. Dazu muß die Liposomensuspension in eine Gel-, Creme- oder Lotionsform, wie oben beschrieben, überfürt werden. Diesen streichfähigen Zubereitungen können natürlich alle oben erwähnten Zusatzstoffe zugesetzt werden.

Folgende Beispiele sollen die Erfindung erläutern, ohne sie jedoch zu beschränken.

### Beispiel 1

Human-α-Interferon enthaltende Liposomen werden auf folgende Weise hergestellt: 0.335 g Lipoid E 100, 0,11 g Cholesterol, 0,055 g PG und 0.5 mg α-Tocopherol werden in 50 ml Chloroform gelöst. 20 mi dieser Lösung werden in einem 100 ml Rundkolben bei Raumtemperatur am Rotavapor eingedampft und 1 Std. bei einem Druck von 0,7-2,0 kPa nachgetrocknet. Es resultiert ein Lipidfilm an der Wand des Rundkolbens.

240 µl Human-α-Interferon (6000000 I.E./ml) werden mit 2160 µl eines Inkubationspuffers (1,5 g Serumalbumin, 0,3 g NaH₂PO₄, 1,2 g Na₂HPO₄ und 8,0 g NaCl pro 1 Wasser; entsprechend einer 1:10 Verdünnung) verdünnt 2 mi dieser Lösung werden in den Rundkolben mit dem Lipidfilm gegeben und dieser Film mit Hilfe von 0,6 g Glaskugeln abgezogen. Es bildet sich eine Interferon-haltige Lipidsuspension mit multilamellaren Vesikeln, die in Portionen von 0,5 ml durch eine Polycarbonatmembran mit einer Porenweite von 200 nm mit Hilfe eines Miniextruders extrudiert wird. Man erhält eine Interferon-haltige oligolamellare Liposomensuspension.

Die direkte Analyse der Suspension mit dem ELISA-Test ergibt als Summe des freien und des außen an die Liposomen gebundenen α-Interferon-Anteils einen Wert von 391000 I.E./ml, der verkapselte Anteil beträgt 209000 I.E./ml entsprechend 35 %.

### Beispiel 2

Human-α-Interferon enthaltende Liposomen werden auf folgende Weise hergestellt: 0.338 g Lipoid E 100, 0,112 g Cholesterol, 0,05 g Dimyristoylphosphatidylglycerol und 0.5 mg α-Tocopherol werden in 50 ml Chloroform gelöst. 20 mi dieser Lösung werden in einem 100 ml Rundkolben bei Raumtemperatur am Rotavapor eingedampft und eine Std. bei einem Druck von 0,7-2,0 kPa nachgetrocknet. Es resultiert ein Lipidfilm an der Wand des Rundkolbens.

240 µl Human-α-Interferon (6000000 I.E./ml) werden mit 2160 µl eines Inkubationspuffers (1,5 g Serumalbumin, 0,3 g NaH₂PO₄, 1,2 g Na₂HPO₄ und 8,0 g NaCl pro 1 Wasser; entsprechend einer 1:10 Verdünnung) verdünnt. 2 ml dieser Lösung werden in den Rundkolben mit dem Lipidfilm gegeben und dieser Film mit Hilfe von 0,6 g Glaskugeln abgezogen. Es bildet sich eine Interferon-haltige Lipidsuspension mit multilamellaren Vesikeln, die in Portionen von 0,5 ml durch eine Polycarbonatmembran mit einer Porenweite von 200 nm mit Hilfe eines Miniextruders extrudiert wird. Man erhält eine Interferon-haltige oligolamellare Liposomensuspension.

Die direkte Analyse der Suspension mit dem ELISA-Test ergibt als Summe des freien und des außen an die Liposomen gebundenen α-Interferon-Anteils einen Wert von 313000 I.E./ml, der verkapselte Anteil beträgt 287000 I.E./ml entsprechend 48 %.

### Beispiel 3 (Vergleichsbeispiel)

Human-α-Interferon enthaltende Liposomen werden auf folgende Weise hergestellt: 0.376 g Lipoid E 100, 0,124 g Cholesterol und 0.5 mg α-Tocopherol werden in 50 ml Chloroform gelöst. 20 ml dieser Lösung werden in einem 100 ml Rundkolben bei Raumtemperatur am Rotavapor eingedampft und eine Std. bei einem Druck von 0,7-2,0 kPa nachgetrocknet. Es resultiert ein Lipidfilm an der Wand des Rundkolbens.

240 µl Human-α-Interferon (6000000 I.E./ml) werden mit 2160 µl eines Inkubationspuffers (1,5 g Serumalbumin, 0,3 g NaH₂PO₄, 1,2 g Na₂HPO₄ und 8,0 g NaCl pro 1 Wasser; entsprechend einer 1:10 Verdünnung) verdünnt. 2 mi dieser Lösung werden in den Rundkolben mit dem Lipidfilm gegeben und dieser Film mit Hilfe von 0,6 g Glaskugeln abgezogen. Es bildet sich eine Interferon-haltige Lipidsuspension mit multilamellaren Vesikeln, die in Portionen von 0,5 ml durch eine Polycarbonatmembran mit einer Porenweite von 200 nm mit Hilfe eines Miniextruders extrudiert wird. Man erhält eine Interferon-haltige oligolamellare Liposomensuspension.

Die direkte Analyse der Suspension mit dem ELISA-Test ergibt als Summe des freien und des außen an die Liposomen gebundenen α-Interferon-Anteils einen Wert von 554000 I.E./ml, der verkapselte Anteil beträgt 46000 I.E./ml entsprechend 8 %.

**Tabelle 1**

| Bsp. | Mengenverhältnis PC/Ch/PG | geb.IFN (I.E./ml) | verkapseltes IFN (I.E./ml) | Einschlußrate (%) |
|---|---|---|---|---|
| 1 | 6:4:1 | 391000 | 209000 | 35 |
| 2 | 6:4:1 | 313000 | 287000 | 48 |
| 3 (Vergleich) | 6:4:0 | 554000 | 46000 | 8 |

In Tabelle 1 sind die Einschlußraten der Beispiele 1 und 2 sowie des Vergleichbeispiels 3 nocheinmal dargestellt. Es ist deutlich zu sehen, daß die Verwendung des erfindungsgemäßen Verfahrens, welches auf einem definierten Mengenverhältnis der die Liposomen konstituierenden Lipidkomponenten beruht, zu einer signifikanten Erhöhung der Einschlußraten führt.

### Beispiel 4

Human-α-Interferon enthaltende Liposomen werden auf folgende Weise hergestellt: 0.336 g Lipoid E 100, 0,111 g Cholesterol, 0,054 g Dipalmitoylphosphatidylglycerol und 0.5 mg α-TocopheroI werden in 50 ml Chloroform gelöst. 20 ml dieser Lösung werden in einem 100 mi Rundkolben bei Raumtemperatur am Rotavapor eingedampft und eine Std. bei einem Druck von 0,7-2,0 kPa nachgetrocknet. Es resultiert ein Lipidfilm an der Wand des Rundkolbens.

240 µl Human-α-Interferon (6000000 I.E./ml) werden mit 2160 µl eines Inkubationspuffers (1,5 g Serumalbumin, 0,3 g NaH₂PO₄, 1,2 g Na₂HPO₄ und 8,0 g NaCl pro 1 Wasser; entsprechend einer 1:10 Verdünnung) verdünnt. 2 ml dieser Lösung werden in den Rundkolben mit dem Lipidfilm gegeben und dieser Film mit Hilfe von 0,6 g Glaskügeln abgezogen. Es bildet sich eine Interferon-haltige Lipidsuspension mit multilamellaren Vesikeln, die in Portionen von 0,5 ml durch eine Polycarbonatmembran mit einer Porenweite von 200 nm mit Hilfe eines Miniextruders extrudiert wird. Man erhält eine Interferon-haltige oligolamellare Liposomensuspension.

### Beispiel 5

Human-α-Interferon enthaltende Liposomen werden auf folgende Weise hergestellt: 0.333 g Lipoid E 100, 0,11 g Cholesterol, 0,057 g Distearoylphosphatidylglycerol und 0.5 mg α-Tocopherol werden in 50 ml Chloroform gelöst. 20 ml dieser Lösung werden in einem 100 ml Rundkolben bei Raumtemperatur am Rotavapor eingedampft und eine Std. bei einem Druck von 0,7-2,0 kPa nachgetrocknet. Es resultiert ein Lipidfilm an der Wand des Rundkolbens.

240 µl Human-α-Interferon (6000000 I.E./ml) werden mit 2160 µl eines Inkubationspuffers (1,5 g Serumalbumin, 0,3 g NaH₂PO₄, 1,2 g Na₂HPO₄ und 8,0 g NaCl pro 1 Wasser; entsprechend einer 1:10 Verdünnung) verdünnt. 2 ml dieser Lösung werden in den Rundkolben mit dem Lipidfilm gegeben und dieser Film mit Hilfe von 0,6 g Glaskugeln abgezogen. Es bildet sich eine Interferon-haltige Lipidsuspension mit multilamellaren Vesikeln, die in Portionen von 0,5 ml durch eine Polycarbonatmembran mit einer Porenweite von 200 nm mit Hilfe eines Miniextruders extrudiert wird. Man erhält eine Interferon-haltige oligolamellare Liposomensuspension.

### Beispiel 6

Zu 1970 µl einer gemäß Beispiel 1,2,4 oder 5 hergestellten Liposomensuspension werden 30 mg Natriumcarboxymethylcellulose (Tylopur C 300 P, eingetragenes Warenzeichen) zugesetzt und der Ansatz 6 Std. quellen gelassen. Es entsteht ein auf der Haut streichfähiges Gel.

### Beispiel 7

Zu 1960 µl einer gemäß Beispiel 1,2,4 oder 5 hergestellten Liposomensuspension werden 40 mg Hydroxyethylcellulose (Tylose H 300, eingetragenes Warenzeichen) zugesetzt und der Ansatz 6 Std. quellen gelassen. Es entsteht ein auf der Haut streichfähiges Gel.

### Beispiel 8

Zu 1750 µl einer gemäß Beispiel 1,2,4 oder 5 hergestellten Liposomensuspension werden 250 mg Hydroxypropylmethylcellulose (Pharmacoat 606, eingetragenes Warenzeichen) zugesetzt und der Ansatz 12 Std. quellen gelassen. Es entsteht ein auf der Haut streichfähiges Gel.

### Beispiel 9

Zu 1970 µl einer gemäß Beispiel 1,2,4 oder 5 hergestellten Liposomensuspension werden 30 mg Natriumalginat (Kelgin F, eingetragenes Warenzeichen) zugesetzt und der Ansatz 6 Std. quellen gelassen. Es entsteht ein auf der Haut streichfähiges Gel.

### Beispiel 10

Die Stabilität der Liposomen wurde anhand ihres Interferongehaltes bestimmt. Die Liposomen wurden gemäß Beispiel 2 und 3 (Vergleichsbeispiel) hergestellt und anschließend für die angegebene Zeit bei 4 °C gelagert. Der Interferongehalt wurde über übliche Verfahren bestimmt.

**Tabelle 2**

| Lagerzeit (Tage) | Beispiel 2 Interferon | | Lagerzeit (Tage) | Beispiel 3 (Vergleich) Interferon | |
|---|---|---|---|---|---|
| | (I.U./ml) | (%) | | (I.U./ml) | (%) |
| 0 | 600000 | 100 | 0 | 600000 | 100 |
| 3 | 519419 | 86,6 | 2 | 452207 | 75,4 |
| 6 | 559241 | 93,2 | 6 | 400011 | 66,7 |
| 11 | 575890 | 96,0 | | | |
| 18 | 614076 | 102,3 | 16 | 383082 | 63,8 |
| 25 | 567961 | 94,7 | 30 | 326904 | 54,5 |
| 34 | 505472 | 84,2 | | | |
| 52 | 576860 | 96,1 | 42 | 314071 | 52,3 |

Aus Tabelle 2 geht deutlich hervor, daß die erfindungsgemäße Verwendung von PG als Ladungsträger das in den Liposomen verkapselte Interferon über einen längeren Zeitraum zu stabilisieren vermag als dies nach dem Stand der Technik (Beispiel 3) möglich ist.

## Patentansprüche

1. Liposomen enthaltend darin verkapselte Proteine, dadurch gekennzeichnet, daß die Liposomen aus Phosphatidylcholin, Cholesterol, Phosphytidylglycerol und Tocopherolen in einem molaren Verhaltnis von 6 zu 4 zu 1 zu 0,01 bestehen.

2. Liposomen gemäß Anspruch 1, bei denen das Phosphatidylglycerolderivat ein Mono- oder Diester desselben mit einer gesättigen oder ungesättigten C₁₂₋₂₄-Fettsäure ist.

3. Liposomen gemäß Anspruch 2, bei denen das Phosphatidylglycerolderivat dessen Myristoyl-, Stearoyl- oder Palmitoyldiester ist.

4. Liposomen gemäß einem der Ansprüche 1 bis 3, bei denen das verkapselte Protein Oxytocin, Vasopressin, FSH, TSH, LH, ein Tumor Nekrosis Faktor, ein Growth Faktor, ein Plättchen aktivierender Faktor, Streptokinase, Urokinase, Insulin, Calcitonin, Cyclosporin A, ein Interleukin oder Interferon ist.

5. Liposomale Zubereitung enthaltend Liposomen gemäß einem der vorstehenden Ansprüche, welche in Form eines Gels vorliegt und bei der der Gelbildner ein Cellulosederivat oder ein Alginat ist.

6. Liposomale Zubereitung gemäß Anspruch 5, in der der Gelbildner Natriumcarboxymethylcellulose, Hydroxyethylcellulose, Hydroxypropylcellulose, Hydroxypropylmethylcellulose oder Natriumalginat ist.

7. Liposomale Zubereitung enthaltend Liposomen gemäß einem der Ansprüche 1 bis 4, die in Form einer Lösung vorliegt.

8. Zubereitung gemäß Anspruch 7, bei der die Lösung isotonisiert und mit einem pharmazeutisch geeigneten Puffer auf pH 7,4 eingestellt ist.

9. Liposomale Zubereitung gemäß einem der Ansprüche 7 und 8, die aseptisch zubereitet und durch eine Membranfiltration sterilisiert ist.

10. Verfahren zur Herstellung von Liposomen, die darin verkapselte Proteine enthalten, welches die folgenden Schritte umfaßt:
a) Lösen von Phosphatidylcholin, Cholesterol, Phosphatidylglycerol und Tocopherol in einem molaren Mengenverhältnis von 6 zu 4 zu 1 zu 0,01 in einem geeigneten Lösungsmittel, Abdampfen des Lösungsmittels und Trocknen des Lipidfilms, und
b) Zugabe des Proteins in einer pharmazeutisch geeigneten Lösung, Ablösen der Liposomen mittels Glaskugeln und anschließender Membranextrusion der Liposomen.

11. Verfahren gemäß Anspruch 10, in dem das Phosphatidylglycerolderivat ein Mono- oder Diester desselben mit einer gesättigten oder ungesättigten C₁₂₋₂₄-Fettsäure ist.

12. Verfahren gemäß Anspruch 11, in dem das Phosphatidylglycerolderivat dessen Myristoyl-, Stearoyl- oder Palmitoyldiester ist.

13. Verfahren gemäß einem der Ansprüche 10 bis 12, in dem das verkapselte Protein Oxytocin, Vasopressin, FSH, TSH, LH, ein Tumor Nekrosis Faktor, ein Growth Faktor, ein Plättchen aktivierender Faktor, Streptokinase, Urokinase, Insulin, Calcitonin, Cyclosporin A, ein Interleukin oder Interferon ist.

14. Verfahren gemäß einem der vorstehenden Ansprüche 10 bis 13, bei dem eine pharmazeutische oder kosmetische Zubereitung in streichfähiger Form erhalten wird, indem die aus Schritt (b) erhaltenen Liposomen mittels bekannter Verfahren unter Verwendung bekannter Träger- und Hilfsstoffe in eine pharmazeutisch und kosmetisch geeignete Form wie ein Gel, eine Creme oder eine Lotion überfürt wird, wobei der Gelbildner ein Cellulosederivat oder ein Alginat ist.

15. Verfahren gemäß Anspruch 14, bei dem der Gelbildner Natriumcarboxymethylcellulose, Hydroxyethylcellulose, Hydroxypropylcellulose, Hydroxypropylmethylcellulose oder Natriumalginat ist.

16. Verfahren gemäß einem der vorstehenden Ansprüche 10 bis 13, bei dem eine pharmazeutische Zubereitung erhalten wird, die in Form einer Lösung vorliegt, indem die aus Schritt (b) erhaltenen Liposomen mittels bekannter Verfahren unter Verwendung bekannter Träger- und Hilfsstoffe in eine Lösung überführt werden.

17. Verfahren gemäß Anspruch 16, bei dem die Lösung mit NaCl isotoniert und mit einem Puffer auf pH 7,4 eingestellt ist.

18. Verfahren gemäß einem der Ansprüche 16 und 17, bei dem die pharmazeutische Zubereitung aseptisch zubereitet und durch eine Membranfiltration sterilisiert ist.

## Claims

1. Liposomes containing proteins encapsulated within them, characterised in that the liposomes consist of phosphatidylcholine, cholesterol, phosphatidylglycerol and tocopherols in a molar ratio of 6 to 4 to 1 to 0.01.

2. Liposomes according to Claim 1 in which the phosphatidylglycerol derivative is a mono- or di-ester of the latter with a saturated or unsaturated C12-24 fatty acid.

3. Liposomes according to Claim 2 in which the phosphatidylglycerol derivative is its myristoyl, stearoyl or palmitoyl di-ester.

4. Liposomes according to one of the Claims 1 to 3 in which the encapsulated protein is oxytocin, vasopressin, FSH, TSH, LH, a tumour necrosis factor, a growth factor, a platelet-activating factor, streptokinase, urokinase, insulin, calcitonin, cyclosporin A, an interleukin or interferon.

5. Liposomal preparation containing liposomes according to one of the preceding Claims, which is present in the form of a gel and in which the gelling agent is a cellulose derivative or an alginate.

6. Liposomal preparation according to Claim 5 in which the gelling agent is sodium carboxymethylcellulose, hydroxyethylcellulose, hydroxypropylcellulose, hydroxypropylmethylcellulose or sodium alginate.

7. Liposomal preparation containing liposomes according to one of the Claims 1 to 4, which is present in the form of a solution.

8. Preparation according to Claim 7 in which the solution is isotonised and adjusted to pH 7,4 with a pharmaceutically suitable buffer.

9. Liposomal preparation according to one of the Claims 7 and 8 which is prepared aseptically and sterilised by membrane filtration.

10. Process for manufacturing liposomes containing proteins encapsulated within them, which comprises the following steps:
a) Dissolving phosphatidylcholine, cholesterol, phosphatidylglycerol and tocopherols in a molar ratio of 6 to 4 to 1 to 0.01 in a suitable solvent, evaporating off the solvent and drying the lipid film, and
b) Addition of the protein in a pharmaceutically suitable solution, removing the liposomes by the use of glass spheres and subsequent membrane-extrusion of the liposomes.

11. Process according to Claim 10 in which the phosphatidylglycerol derivative is a mono- or di-ester of the latter with a saturated or unsaturated C 12-24 fatty acid.

12. Process according to Claim 11 in which the phosphatidylglycerol derivative is its myristoyl, stearoyl or palmitoyl di-ester.

13. Process according to one of the Claims 10 to 12 in which the encapsulated protein is oxytocin, vasopressin, FSH, TSH, LH, a tumour necrosis factor, a growth factor, a platelet-activating factor, streptokinase, urokinase, insulin, calcitonin, cyclosporin A, an interleukin or interferon.

14. Process according to one of the preceding Claims 10 to 13 in which a pharmaceutical or cosmetic preparation in spreadable form is obtained by converting the liposomes obtained from step (b) into a pharmaceutically and cosmetically suitable form such as a gel, a cream or a lotion by the use of known processes using known carriers and adjuvants, whereby the gelling agent is a cellulose derivative or an alginate.

15. Process according to Claim 14 in which the gelling agent is sodium carboxymethylcellulose, hydroxyethylcellulose, hydroxypropylcellulose, hydroxypropylmethylcellulose or sodium alginate.

16. Process according to one of the preceding Claims 10 to 13 in which there is obtained a pharmaceutical preparation that is present in the form of a solution, by converting the liposomes obtained from step (b) into a solution by the use of known processes using known carriers and adjuvants.

17. Process according to Claim 16 in which the solution is isotonised with NaCl and adjusted to pH 7.4 with a buffer.

18. Process according to one of the Claims 16 and 17 in which the pharmaceutical preparation is prepared aseptically and sterilised by membrane filtration.

## Revendications

1. Liposomes contenant des protéines encapsulées, caractérisés par le fait que les liposomes sont constitués par de la phosphatidylcholine, du cholestérol, du phosphatidylglycérol et du tocophérol dans un rapport molaire de 6/4/1/0,01.

2. Liposomes selon la revendication 1, dans lesquels le dérivé de phosphatidylglycérol est un mono- ou diester de celui-ci, avec un acide gras saturé ou insaturé, en C₁₂₋₂₄.

3. Liposomes selon la revendication 2, dans lesquels le dérivé de phosphatidylglycérol est l'ester de myristyle, de stéaryle ou de palmityle de celui-ci.

4. Liposomes selon l'une quelconque des revendications 1 à 3, dans lesquels la protéine encapsulée est l'oxytoxine, la vasopressine, la FSH, la TSH, la LH, un facteur de nécrose tumorale, un facteur de croissance, un facteur activateur de plaquettes, la streptokinase, l'urokinase, l'insuline, la calcitonine, la cyclosporine A, une interleukine ou un interféron.

5. Préparation liposomale contenant des liposomes selon l'une quelconque des revendications précédentes qui est sous forme de gel et dans laquelle le gélifiant est un dérivé cellulosique ou un alginate.

6. Préparation liposomale selon la revendication 5, dans laquelle le gélifiant est la carboxyméthylcellulose sodique, l'hydroxyéthylcellulose, l'hydroxypropylcellulose, l'hydroxyropylméthylcellulose ou l'alginate de sodium.

7. Préparation liposomale contenant des liposomes selon l'une quelconque des revendications 1 à 4, qui est sous la forme d'une solution.

8. Préparation liposomale selon la revendication 7, dans laquelle la solution est rendue isotonique et son pH est ajusté à 7,4 à l'aide d'un tampon pharmaceutique approprié.

9. Préparation liposomale selon l'une des revendications 7 et 8, qui a été préparée dans des conditions aseptiques et stérilisée par une filtration sur membrane.

10. Procédé de préparation liposomale contenant des protéines encapsulées dans les liposomes, comprenant les étapes consistant :
a) à dissoudre la phosphatidylcholine, le cholestérol, le phosphatidylglycérol et le tocophérol dans un rapport molaire de 6/4/1/0,01, dans un solvant approprié, à évaporer le solvant et à sécher le film lipidique, et
b) à introduire la protéine dans une solution pharmaceutique appropriée, à détacher les liposomes à l'aide de billes de verre et ensuite, à extruder les liposomes à travers une membrane.

11. Procédé selon la revendication 10, dans lequel le dérivé de phosphatidylglycérol est un mono- ou diester de celui-ci, avec un acide gras saturé ou insaturé en C₁₂₋₂₄.

12. Procédé selon la revendication 11, dans lequel le dérivé de phosphatidylglycérol est l'ester de myristyle, de stéaryle ou de palmityle de celui-ci.

13. Procédé selon l'une quelconque des revendications 10 à 12, dans lequel la protéine encapsulée est l'oxytoxine, la vasopressine, la FSH, la TSH, la LH, un facteur de nécrose tumorale, un facteur de croissance, un facteur activateur de plaquettes, la streptokinase, l'urokinase, l'insuline, la calcitonine, la cyclosporine A, une interleukine ou un interféron.

14. Procédé selon l'une quelconque des revendications 10 à 13, dans lequel on obtient une préparation pharmaceutique ou cosmétique sous forme facile à étaler, dans lequel les liposomes obtenus dans l'étape (b) sont transformés, par des procédés connus, en utilisant des véhicules et adjuvants connus, en une forme pharmaceutique et cosmétique appropriée telle qu'un gel, une crème ou une lotion, le gélifiant étant un dérivé cellulosique ou un alginate.

15. Procédé selon la revendication 14, dans lequel le gélifiant est la carboxyméthylcellulose sodique, l'hydroxyéthylcellulose, l'hydroxypropylcellulose, l'hydroxypropyl-méthylcellulose ou l'alginate de sodium.

16. Procédé selon l'une quelconque des revendications 10 à 13, dans lequel on obtient une préparation pharmaceutique qui se présente sous forme de solution, les liposomes obtenus dans l'étape (b) ayant été amenés sous la forme d'une solution par des procédés connus, en utilisant des véhicules et adjuvants connus.

17. Procédé selon la revendication 16, dans lequel la solution est rendue isotonique par du NaCl et son pH est ajusté à 7,4 avec un tampon.

18. Procédé selon l'une des revendications 16 et 17, dans lequel la préparation pharmaceutique a été préparée dans des conditions aseptiques et stérilisée par une filtration sur membrane.
